# EUROPÄISCHE PATENTSCHRIFT

(11) **EP 1 112 094 B1**
(45) Veröffentlichungstag und Bekanntmachung des Hinweises auf die Patenterteilung: **25.06.2003**
(21) Anmeldenummer: 99941632.4
(22) Anmeldetag: 18.08.1999
(51) Int. Cl.: A61L 27/18

(54) **BESCHICHTETE MEDIZINISCHE GERÄTE UND IMPLANTATE**
COATED MEDICAL DEVICES AND IMPLANTS
APPAREILS ET IMPLANTS MEDICAUX PRESENTANT UN REVETEMENT

(30) Priorität: 10.09.1998 DE 19843254
(43) Veröffentlichungstag der Anmeldung: 04.07.2001
(73) Patentinhaber: SCHERING AKTIENGESELLSCHAFT, 13342 Berlin (DE)
(72) Erfinder: PRIEWE, Jörg, D-13347 Berlin (DE); HELDMANN, Dieter, D-10555 Berlin (DE); WINDT-HANKE, Fred, D-13435 Berlin (DE); BERNDORFF, Dietmar, D-16540 Hohen Neuendorf (DE)
(86) Internationale Anmeldenummer: EP9906015
(87) Internationale Veröffentlichungsnummer: WO00015270

(56) Entgegenhaltungen:
- EP-A- 0 727 230
- WO-A-94/14390
- DE-A- 1 928 104
- GB-A- 1 196 049
- US-A- 5 792 106
- DATABASE WPI Section Ch, Week 199515 Derwent Publications Ltd., London, GB; Class A96, AN 1995-109588 XP002124006 & JP 07 031673 A (ASAHI OPTICAL CO LTD), 3. Februar 1995 (1995-02-03)

## Beschreibung

Die Erfindung liegt auf dem Gebiet der Beschichtung medizinischer Geräte und Implantate, welche zur Behandlung proliferativer Erkrankungen wie z.B. Tumoren oder Erkrankungen aus dem atherosklerotischen Forrnenkreis eingesetzt werden.

Herz/Kreislauferkrankungen sind weitverbreitete Krankheiten in den Industrienationen. Sie stellen eine der häufigsten Todesursachen dar. In den allermeisten Fällen werden Herz/Kreislauferkrankungen durch die Atherosklerose hervorgerufen. Diese ist eine entzündliche, fibroproliferative Erkrankung, die für 50% aller Todesfälle in den USA, Europa und Japan verantwortlich ist (Ross 1993, Nature 362: 801-809). Mit ihrer peripheren Ausprägung bedroht sie den Erhalt der Extremitäten, mit ihrer koronaren Manifestation besteht das Risiko des tödlichen Herzinfarkts und mit supraaortalem Befall droht der Schlaganfall.

Eine Behandlung der Atherosklerose erfolgt derzeit auf unterschiedlichen Wegen. So hat sich neben den konservativen Maßnahmen (z. B. die Senkung des Cholesterinspiegels im Blut) und der Bypass-Operation, auch die mechanische Dilatation (Angioplastie) sowie die intravasale Entfernung atheromatösen Gewebes (Atherektomie) verengter Segmente in peripheren Arterien und den Koronarien als Alternative im klinischen Alltag etabliert.

Wie nachfolgend ausgeführt, sind die genannten Methoden jedoch mit einer Vielzahl von Nachteilen behaftet.

So wird der Wert mechanisch rekanalisierender Verfahren akut durch Gefäßverschlüsse in Folge von Gefäßeinrissen und -dissektionen sowie akuten Thrombosen beeinträchtigt (Sigwart et al. 1987, N. Engl. J. Med. 316: 701-706). Der langfristige Erfolg wird durch das Wiederauftreten von Einengungen (Restenosen) gefährdet. So ergab die CAVEAT-Studie an 1012 Patienten, daß die Restenoserate sechs Monate nach Intervention bei der koronaren Atherektomie 50% und bei der koronaren Angioplastie sogar 57% betrug (Topol et al. 1993, N. Engl. J. Med. 329: 221-227). Weiterhin traten in dieser Studie in 7% der Atherektomie- und in 3% der Angioplastie-Patienten abrupte Gefäßverschlüsse auf. Nicolini und Pepine (1992, Endovascular Surgery 72: 919-940) berichten von einer Restenoserate zwischen 35 und 40% und einer akuten Verschlußrate von 4% nach angioplastischen Eingriffen.

Um diesen Komplikationen zu begegnen, wurden verschiedene Techniken entwickelt. Hierzu gehört die Implantation metallischer Endoprothesen (Stents), (Sigwart et al. 1987, N. Engl. J. Med. 316: 701-706; Strecker et al., 1990, Radiology 175: 97-102). Die Stentimplantation in großkalibrigen Arterien, z.B. bei Okklusionen in der Beckenachse hat bereits den Rang einer primär anzuwendenden Therapiemodalität erhalten. Der Einsatz von Stents in den Femoralarterien hat dagegen mit einer primären Offenheitsrate von 49% und einer Reokklusionshäufigkeit von 43% enttäuschende Ergebnisse gezeigt (Sapoval et al., 1992, Radiology 184: 833-839). Ebenfalls unbefriedigende Resultate hauptsächlich bedingt durch Restenose, wurden mit bisher verfügbaren Stents in den Koronararterien erzielt (Kavas et al. 1992, J. Am. Coll. Cardiol 20: 467-474).

Alle bisherigen pharmakologischen und mechanischen Interventionen haben bis heute die Restenose nicht verhindern können (Muller et al. 1992, J. Am. Coll. Cardiol. 19: 418-432).

Als Ursache für die nach mechanischen Eingriffen häufig auftretenden Restenosen wird angenommen, daß die Eingriffe eine Proliferation und Migration glatter Muskelzellen in der Gefäßwand induzieren. Diese führen zu einer neointimalen Hyperplasie und den beobachteten Restenosen in den behandelten Gefäßabschnitten (Cascells 1992, Circulation 86: 723-729, Hanke et al. 1990, Circ. Res. 67: 651-659, Ross 1993, Nature 362: 801-809).

Ein alternatives Verfahren zur Behandlung von atherosklerotischen Erkrankungen wird von Sonobe et al. beschrieben (Sonobe et al., The International Journal of Artificial Organs, Vol. 20 (6): 1997, 319 - 326). Dieses Verfahren wird "Intracoronary Local Adhesive Delivery Technique" genannt, dabei handelt es sich um die lokale Applikation adhäsiver Agenzien an der Stelle der atherosklerotischen Läsion. Bevorzugt wird Cyanacrylat-Monomer an die Stelle der Läsion gebracht, das dort polymerisiert und im günstigsten Fall einen steifen Tunnel entlang der Arterienwand bildet. Diese Methode hat allerdings wesentliche Nachteile. Sie erfordert einerseits sehr viel Geschick des behandelnden Arztes, da Cyanacrylate in Gegenwart von Feuchtigkeit sehr schnell polymerisieren können. Daher muß sehr auf eine schnelle, sichere und trockene Arbeitsweise geachtet werden. Andererseits besteht die Gefahr, daß sich bereits polymerisiertes Cyanacrylat wieder von der Arterienwand löst oder kurz nach der Applikation ein Teil des Cyanacrylats abgeschwemmt wird, das sich später im feinen Geäst der Herzkranzgefäße festsetzt und somit einen Herzinfarkt auslösen kann. Weiter ist aus der Literatur seit langem bekannt, daß das monomere Cyanacrylat das Gewebe reizt (Tseng et al., Medical Application of Cyanoacrylates as Surgical Adhesives, Japanese Journal of Artificial Organs; 18(1): 409-413; 1989).

Es besteht daher die Aufgabe, medizinische Geräte und Implantate zur Verfügung zu stellen, die bei der Behandlung proliferativer Erkrankungen, wie z.B. der Atherosklerose oder Tumoren, eingesetzt werden können und mit Hilfe derer die Nachteile des Standes der Technik überwunden werden.

Diese Aufgabe wird durch die medizinischen Implantate gelöst, die in den Patentansprüchen beschrieben sind.

Es wurde gefunden, daß medizinische Implantate, die mit polymeren Gemischen beschichtet sind, welche Polymere aus Cyanacrylat (Polycyanacrylsäureester) oder Methylenmalonsäureester enthalten, überraschenderweise die Proliferation von glatten Muskelzellen oder Tumorzellen verhindern und somit zur Restenoseprophylaxe hervorragend geeignet sind. Besonders überraschend war hierbei der Befund, daß - wie in den Beispielen eindrucksvoll gezeigt - sehr kleine Mengen von polymerem Cyanacrylat ausreichen, um einen deutlichen antiproliferativen Effekt in den beiden untersuchten Zellkultur-Modellen (Tumorzellen und glatte Muskelzellen) zu bewirken.

Die oben genannten Nachteile bei der lokalen Applikation von Cyanacrylat-Monomer und anschließender Polymerisation im Blutgefäß treten bei den erfindungsgemäßen Implantaten nicht auf, da das Cyanacrylat nicht in monomerer, sondern in polymerer Form eingesetzt wird. Hierdurch ist sichergestellt, daß es bei der Verwendung der erfindungsgemäßen Implantate nicht zur unerwünschten Polymerbildung abseits der vorgesehenen Applikationsstelle kommen kann. Desweiteren treten die literaturbekannten Gewebereizungen durch das Monomer bei Verwendung der erfindungsgemäßen Implantate nicht auf. Darüberhinaus sind mögliche Anwendungsprobleme aufgrund der spontanen Neigung von Cyanacrylatmonomer zur Polymerisation in Anwesenheit von Feuchtigkeit - wie zum Beispiel das Verkleben der Applikationsbestecke - bei den erfindungsgemäßen Implantaten nicht möglich. Weiter ist die Haftung des Polymeren an der Oberfläche der Implantate wesentlich besser als an der Körperoberfläche der Implantationsstelle (z.B. eine luminale Arterienoberfläche). Dadurch wird das Embolierisiko durch sich ablösende Polymerteile vermieden.

Die Herstellung der erfindungsgemäßen Implantate erfolgt beispielsweise dadurch, daß ein Träger oder ein Implantat, wie z.B. ein Stent, oder der zu beschichtende Teil eines medizinischen Implantates in eine das Polymer enthaltende Lösung getaucht wird. Das Polymer bleibt nach dem Herausziehen auf dem Träger oder dem Implantat haften und trocknet an der Luft. Diese Art der Herstellung hat den Vorteil, daß die Beschichtung des Implantats unmittelbar vor der Implantation nach den Bedürfnissen des Patienten vom Arzt selbst ausgeführt werden kann. Zur besonders bequemen Handhabbarkeit kann die sterile Polymerlösung in einem speziellen Inkubationsgefäß als "Pre-Application-Kit" zur Verfügung gestellt werden.

Eine weitere Variante zur Herstellung der erfindungsgemäßen Implantate ist die CVD-Technik (Chemical Vapour Deposition). Dabei wird das Cyanacrylat und/oder der Methylenmalonsäureester auf den Träger aufgedampft.

Als Träger kommen die handelsüblichen Stents infrage, wie z.B. ein Wiktor-Stent, ein Palmaz-Schatz-Stent oder ein Strecker-Stent. Die Stents können aus Metall (z.B. Nirosta-Stahl) oder einem Polymer bestehen (z.B. aus Polyethylenterephtalat, Silikon, Polyurethanharnstoff). Es ist auch möglich, Katheter und andere medizinische Geräte mit Polymeren, welche Cyanacrylat und/oder Methylenmalonsäureester enthalten, zu beschichten.

Die auf den Träger aufgebrachte Polymerschicht sollte zwischen 5 µm und 200 µm liegen. Bevorzugt sind Schichtdicken zwischen 20 µm und 150 µm.

Cyanacrylat oder Methylenmalonsäureester können ausschließlich zur Polymerisation und anschließender Beschichtung verwendet werden. Besonders bevorzugt wird n-Butyl-2-cyanoacrylat oder Cyanacrylatbutylester zur Polymerisation verwendet.

Es ist auch möglich, eines der Polymere aus Cyanacrylat oder Methylenmalonsäureester zusammen mit anderen Polymeren auf den Träger aufzubringen, wobei die anderen Polymere aus einer der nachfolgenden Substanzgruppen stammen:
Proteine (insbesondere Albumin, Gelatine, Fibrinogen, Fibrin, Hirudin, Heparin, Collagen, oder Immunoglobuline) sowie deren Derivate (insbesondere quervemetzte Polypeptide, Konjugate von Proteinen mit Polyethylenglykolen und anderen Polymeren), Pseudopolyaminosäuren, Stärke oder Stärkederivate, Chitin, Chitosan, Pektin, Polymilchsäure, Polyglykolsäure, Polyhydroxybuttersäure, Polyester, Polycarbonate, Polyamide, Polyphosphazene, Polyvinylalkohol, Polyaminosäuren, Poly-ε-caprolacton, Polyorthoester, Polyurethane, Polyharnstoff, Polyethylenterephtalat.

Weiter kann auch ein Polymergemisch aus Cyanacrylat und Methylenmalonsäureester zur Beschichtung verwendet werden.

Bei all diesen Polymermischungen sollte der Anteil an Cyanacrylat bzw. Methylenmalonsäureester in der auf das Implantat aufgebrachten Polymerschicht zwischen 100% und 10% liegen. Bevorzugt ist ein Gehalt an Cyanacrylat bzw. Methylenmalonsäureester von 100% bis 75%. Besonders bevorzugt ist ein Gehalt an Cyanacrylat bzw. Methylenmalonsäureester von 100% bis 80%.

Das Molekulargewicht der verwendeten Polymere liegt im Bereich von 1.000 bis 10.000.000 Dalton. Durch das Molekulargewicht des Polymeren kann dessen Abbaugeschwindigkeit gesteuert werden. Desweiteren können auch Substanzen, die den Abbau des Polymeren beeinflussen, wie z.B. Kalziumcarbonat, in der Polymermischung enthalten sein.

Weiter können die Polymergemische noch andere Zusatzstoffe wie z.B. Weichmacher enthalten. Beispiele für Weichmacher sind u.a. nichtionische Tenside wie Nonylphenoxy-Polyethylenoxid (Synperonic NP20), Octoxynol (Triton X-100) oder Poloxamere (insbesondere Pluronic F127 oder Pluronic F68).

Die nachfolgenden Beispiele sollen den Erfindungsgegenstand verdeutlichen, ohne ihn auf diese beschränken zu wollen.

### Beispiel 1:

### Herstellung von Polybutylcyanacrylat durch Polymerisation an Grenzflächen.

Auf den Boden einer Kristallisierschale aus Glas (d = 47 cm) werden 5g Butylcyanoacrylat (Sichel, Lot.No. 82902065) durch mehrmaliges Schwenken gleichmäßig verteilt. Das Polymerisat wird 2 Tage offen stehengelassen und dann in THF durch leichtes Erwärmen gelöst.

### Beispiel 2:

### Herstellung von Polybutylcyanacrylat durch Polymerisation in Ethanol / Wasser.

Zu 100 ml Ethanol/Wasser (50 %ig) werden 4 ml Butylcyanoacrylat (Sichel,Lot.No. 82902065) mit einer Spritze unter Rühren zugegeben.
Nach zwei Stunden werden 100 ml Wasser zugesetzt und das Polymer über eine Glasfritte filtriert und an der Luft getrocknet. Das Pulver wird mit etwa 50 ml Methylenchlorid versetzt und gelöst. Das Methylenchlorid und der restlicher Alkohol bzw. Wasser wird durch Eindampfen bei 50 °C entfernt. (Ausbeute 3.6 g)

### Beispiel 3:

Zur Beschichtung einer Probe aus "medical grade" Nirosta-Stahl mit dem nach Beispiel 1 hergestellten Polymer wird wie folgt vorgegangen:
1.) 50 mg Poly-2-cyanoacrylsäurebutylester hergestellt nach Beispiel 1werden in einem ml des Lösemittels Tetrahydrofuran (THF) durch 2 Stunden Rühren bei 40° Celsius gelöst.
2.) Die mit THF gereinigte Probe des "medical grade" Nirosta-Stahls" wird mit einer Geschwindigkeit von einem cm pro Sekunde durch die Öffnung des Beschichtungsapparates gezogen, der die o.g. Polymerlösung enthält. Hierbei scheidet sich ein dünner Film Polymerlösung auf der Oberfläche der Probe ab.
3.) Nach dem Evaporieren des Lösemittels aus der abgeschiedenen Polymerlösung (Inkubation bei Raumtemperatur für 12 h) bleibt auf der Probe ein dünner Film aus Poly-2-cyanoacrylsäurebutylester zurück.
4.) Eine lichtmikroskopische Untersuchung ergibt eine Schichtdicke des abgeschiedenen Poly-2-cyanoacrylsäurebutytesterfilmes von ca. 30 µm.

### Beispiel 4:

Die therapeutische Wirksamkeit einer nach Beispiel 3 mit Poly-2-cyanoacrylsäurebutylester beschichteten "medical grade" Nirosta-Stahl- Probe wird wie folgt gezeigt:
1.) LS174T-Zellen (Tumorzellen) werden in einer Standard Kulturschale in Kultur gebracht (DMEM-Medium mit 10% fotalem Kälberserum; 37° Celsius; 5% Kohlendioxid). Dieser Ansatz dient als Kontrolle **ohne** mit Poly-2-cyanoacrylsäurebutytester beschichtete "medical grade" Nirosta-Stahl-Probe.
2.) Eine nach Beispiel 1 mit Poly-2-cyanoacrylsäurebutytester beschichtete "medical grade" Nirosta-Stahl-Probe von 3 cm Länge wird mit einem sich extern an der Kulturschale befindlichem Magneten am Boden derselben fixiert. Danach wurden die kultivierten LS174T-Zellen in diese Kulturschale überführt.
3.) Analog zu 2.) wurde eine nicht mit Poly-2-cyanoacrylsäurebutytester beschichtete "medical grade" Nirosta-Stahl-Probe in ein Kulturgefäß eingebracht und dort magnetisch fixiert. Danach wurden die kultivierten LS174T-Zellen in diese Kulturschale überführt. Dieser Ansatz diente als Kontrolle **mit** "medical grade" Nirosta-Stahl-Probe, jedoch **ohne** Beschichtung derselben mit Poly-2-cyanoacrylsäurebutytester.

Der Zustand der Zellen in den Ansätzen 1.) bis 3.) wurde nach 24 h, 48 h und 72 h kontrolliert. Als Kriterium zur Bewertung des Zustandes der Zellkulturen diente die Ausbildung eines homogenen Zellrasens auf dem Boden der Kulturflaschen.

Es ergaben sich folgende Befunde:
In dem unter 1.) beschriebenen Ansatz vermehrten sich die Zellen normal und bildeten einen "nahezu homogenen" Zellrasen.
In dem unter 2.) beschriebenen Ansatz war der Zustand der Zellkultur nach 24 h deutlich schlechter. Es hatte sich kein "nahezu homogener" Zellrasen gebildet. Auch nach 48 und 72 h waren noch keine Zellen angewachsen.
In dem unter 3.) beschriebenen Ansatz war der Zustand der Zellkultur wie in der unter 1.) beschriebenen Kontrolle.

Diese Untersuchung zeigt deutlich, daß das Beschichten der "medical grade" Nirosta-Stahl-Probe mit Poly-2-cyanoacrylsäurebutytester das Wachstum der Zellen verhindert.

### Beispiel 5:

Die therapeutische Wirksamkeit einer dünnen, nach Beispiel 2 hergestellten, auf die innere Oberfläche einer Glasschale aufgebrachten Schicht Poly-2-cyanoacrylsäurebutylester wird wie folgt gezeigt:
1.) A10-Zellen (glatte Muskelzellen) werden in einer sterilen Glasschale (Durchmesser 4 cm; Höhe 1.5 cm) in Kultur gebracht (30.000 Zellen pro Ansatz; DMEM-Medium mit 10% fötalem Kälberserum; 37° Celsius; 5% Kohlendioxid). Dieser Ansatz dient als Kontrolle **ohne** Poly-2-cyanoacrylsäurebutytester.
2.) Aus einer Lösung von 0.4 % (w/w) Poly-2-cyanoacrylsäurebutytester in THF werden 0.5 ml in eine Glasschale pipettiert. Das THF wird bei Raumtemperatur evaporiert, um einen dünnen Film aus 1,8 mg Poly-2-cyanoacrylsäurebutytester in der Glasschale abzuscheiden. Danach wurden die kultivierten A10-Zellen in diese Kulturschale überführt.
3.) Aus einer Lösung von 0,04 % (w/w) Poly-2-cyanoacrylsäurebutytester in THF werden 0,5 ml in eine Glasschale pipettiert. Das THF wird bei Raumtemperatur evaporiert, um einen dünnen Film aus 0,18 mg Poly-2-cyanoacrylsäurebutytester in der Glasschale abzuscheiden. Danach wurden die kultivierten A10-Zellen in diese Kulturschale überführt.
4.) Aus einer Lösung von 0,004 % (w/w) Poly-2-cyanoacrylsäurebutytester in THF werden 0,5 ml in eine Glasschale pipettiert. Das THF wird bei Raumtemperatur evaporiert, um einen dünnen Film aus 0,018 mg Poly-2-cyanoacrylsäurebutytester in der Glasschale abzuscheiden. Danach wurden die kultivierten A10-Zellen in diese Kulturschale überführt.

Der Zustand der Zellen in den Ansätzen 1.) bis 4.) wurde nach 24 h, 48 h und 72 h kontrolliert. Als Kriterium zur Bewertung des Zustandes der Zellkulturen diente die Anzahl der lebenden und toten Zellen, deren Anzahl lichtmikroskopisch bestimmt wurde.

Es ergaben sich folgende Befunde:
In dem unter 1.) beschriebenen Ansatz sind die Zellen in der Glasschale gut angewachsen. Es hat sich ein "nahezu homogener" Zellrasen gebildet.
In dem unter 2.) und 3.) beschriebenen Ansätzen sind keine Zellen angewachsen.
In dem unter 4.) beschriebenen Ansatz hat sich ein "weniger dichter" Zellrasen (im Vergleich zur Kontrolle 1.) gebildet.

Diese Untersuchung zeigt deutlich, daß mit einer dünnen Schicht Poly-2-cyanoacrylsäurebutytester das Wachstum der A10-Zellen wirkungsvoll und dosisabhängig verhindert werden kann.

### Beispiel 6:

### Beschichtung eines medizinischen Implantates mit einem Polymeren unter Verwendung eines das Polymer enthaltenen "Kits".

Der Kit besteht aus einem Glasvial (25 ml Inhalt; mit wieder verschließbarer Kappe) enthaltend eine 0.6 % (w/w) Poly-2-cyanoacrylsäurebutytester (hergestellt nach Beispiel 1.) in THF. Das medizinische Implantat (ein Stent mit metallischem Grundkörper) wird aus seiner Verpackung entfernt und unter sterilen Bedingungen in das "Kit-Vial" mit der Polymerlösung eingebracht. Das Vial wird verschlossen und mehrfach leicht geschüttelt, um den Stent gleichmäßig mit Polymerlösung zu benetzten. Danach wird der Stent unter sterilen Bedingungen aus dem Vial entnommen und unter sterilen Bedingungen getrocknet. Der Stent ist jetzt mit Polymer beschichtet und einsatzbereit.

### Beispiel 7:

### Zur Beschichtung einer Probe aus "medical grade" Nirosta-Stahl mit dem nach Beispiel 1 hergestellten Polymer wird wie folgt vorgegangen:

Die Polymerlösung befindet sich in einem engen zylinderförmigen Gefäß. Die mit dem Lösemittel THF gereinigten medical grade" Nirosta-Stahl-Proben werden in die moderat viskoser Polymerlösung eingetaucht und nach kurzer Inkubationszeit (ca. 10-15 sek) senkrecht mit ca. 1 cm/Sekunde herausgezogen. Überschüssige Polymerlösung tropft nach unten ab, während ein Flüssigkeitsfilm viskositätsbedingt auf der Oberfläche der medical grade" Nirosta-Stahl-Probe verbleibt. Nach einer Zwischentrocknung unter sterilen Bedingungen an Luft (20-22°C) wird ein zweiter Tauchvorgang in gleicher Weise durchgeführt. Nach erneuter Zwischentrocknung folgt ein dritter und letzter Tauchvorgang, worauf die "medical grade" Nirosta-Stahl-Proben vollständig getrocknet werden. (Inkubation bei Raumtemperatur für 12 h).
Eine sich anschließende lichtmikroskopische Untersuchung ergibt eine Schichtdicke des abgeschiedenen Poly-2-cyanoacrylsäurebutytesterfilmes von ca. 50 µm.

### Beispiel 8:

### Herstellung von Polyethylcyanacrylat durch Polymerisation an Grenzflächen.

Auf den Boden einer großen Kristallisierschale (d = 47 cm) werden 5g Ethylcyanoacrylat (Fa. Sichel) durch schwenken gleichmäßig verteilt. Das Polymerisat wird 2 Tage offen stehengelassen und dann in THF durch leichtes Erwärmen gelöst.

### Beispiel 9:

### Beschichtung eines medizinischen Implantates mit einem Blend aus Polymer und Tensid

Es wird unter sterilen Bedingungen eine Lösung aus Polybutylcyanacrylat (1, 5%-ig) und nichtionischem Tensid (Synperonic NP20, ICI, 0,5 %-ig) in Methylenchlorid hergestellt. Diese Mischung wird zur Beschichtung eines medizinischen Implantates wie in Beispiel 3 beschrieben verwendet.

### Beispiel 10:

### Beschichtung eines medizinischen Implantates mit einem Blend aus Polymer und Tensid

Es wird unter sterilen Bedingungen eine Lösung aus Polybutylcyanacrylat (1, 5%-ig) und nichtionischem Tensid Triton X-100 (0,2%) in Methylenchlorid hergestellt. Diese Mischung wird zur Beschichtung eines medizinischen Implantates wie in Beispiel 3 beschrieben verwendet.

### Beispiel 11:

### Beschichtung eines medizinischen Implantates mit einem Polymerblend aus 2 Polymeren

Es wird unter sterilen Bedingungen eine Lösung aus Polybutylcyanacrylat (1, 5%-ig) und nichtionischem Tensid Pluronic F127 in Methylenchlorid hergestellt. Diese Mischung wird zur Beschichtung eines medizinischen Implantates wie in Beispiel 3 beschrieben verwendet.

### Beispiel 12:

### Beschichtung eines medizinischen Implantates mit einem Polymerblends aus 2 Polymeren

Es wird unter sterilen Bedingungen eine Lösung aus Polybutylcyanacrylat (1, 5%-ig) und nichtionischem Tensid Pluronic F68 in Methylenchlorid hergestellt. Diese Mischung wird zur Beschichtung eines medizinischen Implantates wie in Beispiel 3 beschrieben verwendet.

### Beispiel 13:

### Beschichtung eines medizinischen Implantates mit einem Polymerblend aus 2 Polymeren

Es wird unter sterilen Bedingungen eine Lösung aus Polybutylcyanacrylat (0,3%) und Polylactide-co-glycolide : Resomer RG503 (2,7%; Boehringer Ingelheim) in Methylenchlorid hergestellt. Diese Mischung wird zur Beschichtung eines medizinischen Implantates wie in Beispiel 3 beschrieben verwendet.

### Beispiel 14:

### Beschichtung eines medizinischen Implantates mit einem Polymerblend aus 2 Polymeren

Es wird unter sterilen Bedingungen eine Lösung aus Polybutylcyanacrylat (0,6%) und Poly-L-Lactide Resomer L 104 (2,4%; Boehringer Ingelheim) in Methylenchlorid hergestellt. Diese Mischung wird zur Beschichtung eines medizinischen Implantates wie in Beispiel 3 beschrieben verwendet.

### Beispiel 15:

### Beschichtung eines medizinischen Implantates mit einem Polymerblend aus 2 Polymeren

Es wird unter sterilen Bedingungen eine Lösung aus Polybutylcyanacrylat (0,6%) und Poly-D,L-Lactide Resomer R 503 (3,4%; Boehringer Ingelheim) in THF hergestellt. Diese Mischung wird zur Beschichtung eines medizinischen Implantates wie in Beispiel 3 beschrieben verwendet.

### Beispiel 16:

### Beschichtung eines medizinischen Implantates mit einem Polymerblend aus 2 Polymeren

Es wird unter sterilen Bedingungen eine Lösung aus Polybutylcyanacrylat (6,0%) Polyethylenglycol 5000 (0,18%; Fluka) in Methylenchlorid hergestellt. Diese Mischung wird zur Beschichtung eines medizinischen Implantates wie in Beispiel 3 beschrieben verwendet.

### Beispiel 17:

### Beschichtung eines medizinischen Implantates mit einem Polymerblend aus 2 Polymeren

Es wird unter sterilen Bedingungen 10 ml einer Lösung aus Polybutylcyanacrylat (8%) und einem AB-Blockcopolymer (2%), bestehend aus 98 % Poly-e-caprolactone und 2% Polyethylene glycol 5000 (Birmingham Polymers), in Methylenchlorid hergestellt. Diese Mischung wird zur Beschichtung eines medizinischen Implantates wie in Beispiel 3 beschrieben verwendet.

### Beispiel 18:

### Beschichtung eines medizinischen Implantates mit einem Polymerblend aus 2 Polymeren

Es wird unter sterilen Bedingungen 10 ml einer Lösung aus Polybutylcyanacrylat (8%) und einem AB-Blockcopolymer (2%), bestehend aus 80 % Poly-e-caprolactone und 20% Polyethyleneglycol 5000 (Birmingham Polymers), in Methylenchlorid hergestellt.
Diese Mischung wird zur Beschichtung eines medizinischen Implantates wie in Beispiel 3 beschrieben verwendet.

### Beispiel 19:

### Beschichtung eines medizinischen Implantates mit einem Polymerblend aus 2 Polymeren

Es wird unter sterilen Bedingungen 10 ml einer Lösung aus Polybutylcyanacrylat (8%) und einem AB-Blockcopolymer (2%), bestehend aus 70/30 D,L-Polylactide-coglycolide und PEG5000 (Inherent Visc. = 0.68 dl/g, Birmingham Polymers), in Methylenchlorid hergestellt.
Diese Mischung wird zur Beschichtung eines medizinischen Implantates wie in Beispiel 3 beschrieben verwendet.

### Beispiel 20:

### Beschichtung eines medizinischen Implantates mit einem Polymerblend aus 2 Polymeren

Es wird unter sterilen Bedingungen 10 ml einer Lösung aus Polybutylcyanacrylat (8%) und einem AB-Blockcopolymer (2%), bestehend aus 70/30 D,L-Polylactide-coglycolide und PEG5000 (Inherent Visc. = 0.94 dl/g, Birmingham Polymers), in THF hergestellt.
Diese Mischung wird zur Beschichtung eines medizinischen Implantates wie in Beispiel 3 beschrieben verwendet.

### Beispiel 21:

### Beschichtung eines medizinischen Implantates mit einem Polymerblend aus 2 Polymeren

Es wird unter sterilen Bedingungen 5 ml einer Lösung aus Polybutylcyanacrylat (15%) in Methylenchlorid hergestellt. Zu dieser Lösung werden 5 ml einer weitere Polymerlösung enthaltend PEG-Cyanacrylat hergestellt nach: Perrachia et al. Macromolecules 30: 846-851 (1997) in THF zugegeben. Diese Mischung wird zur Beschichtung eines medizinischen Implantates wie in Beispiel 3 beschrieben verwendet.

### Beispiel 22:

### Beschichtung eines medizinischen Implantates mit einem Polymerblend aus 2 Polymeren

Es wird unter sterilen Bedingungen eine Lösung aus Polybutylcyanacrylat (10%) und Polyvinylakohol (0,85%) in Methylenchlorid hergestellt. Diese Mischung wird zur Beschichtung eines medizinischen Implantates wie in Beispiel 3 beschrieben verwendet.

### Beispiel 23:

### Beschichtung eines medizinischen Implantates mit einem Gemisch aus Polybutylcyanacrylat und einem Phospholipid.

Es wird unter sterilen Bedingungen eine Lösung aus Polybutylcyanacrylat (9,0%) und dem Phospholipid DSPE-PEG5000 (Shearwater Polymers) in Methylenchlorid hergestellt. Diese Mischung wird zur Beschichtung eines medizinischen Implantates wie in Beispiel 3 beschrieben verwendet.

### Beispiel 24:

### Beschichtung eines medizinischen Implantates mit einem Gemisch aus Polybutylcyanacrylat und Palmitinsäure.

Es wird unter sterilen Bedingungen eine Lösung aus 5 g Polybutylcyanacrylat und 0,5 g Palmitinsäure in 100 ml Methylenchlorid hergestellt. Diese Lösung wird zur Beschichtung eines medizinischen Implantates wie in Beispiel 3 beschrieben verwendet.

### Beispiel 25:

### Beschichtung eines medizinischen Implantates mit einer Polymermischung unter Verwendung eines "Kits".

Der Kit besteht aus einem Glasvial (25 ml Inhalt; mit wieder verschließbarer Kappe) enthaltend eine Polymergemischlösung hergestellt nach Beispiel 14 in THF. Das mediznische Implantat (ein Stent mit metallischem Grundkörper) wird aus seiner Verpackung entfernt und unter sterilen Bedingungen in das "Kit-Vial" mit der Polymergmischlösung eingebracht. Das Vial wird verschlossen und mehrfach leicht geschüttelt, um den Stent gleichmäßig mit Polymergemischlösung zu benetzten. Danach wird der Stent unter sterilen Bedingungen aus dem Vial entnommen und unter sterilen Bedingungen getrocknet. Der Stent ist jetzt mit Polymergemisch beschichtet und einsatzbereit.

### Beispiel 26:

### Beschichtung eines medizinischen Implantates mit einem Polymer und einem basischen Hilfsstoff zur Steuerung der Polymerdegradation.

Es wird unter sterilen Bedingungen eine Lösung aus Polybutylcyanacrylat (1,3%) in Methylenchlorid hergestellt. In dieser Lösung werden 0.15 % hydrobisierte CaCO₃ Mikropartikel (Winnofil, Zeneca) mit einem Ultraturrax fein dispergiert. Diese Dispersion wird zur Beschichtung eines medizinischen Implantates wie in Beispiel 3 beschrieben verwendet.

### Beispiel 27:

### Beschichtung eines medizinischen Implantats mit einem Polymer

Es wird ein Methylenmalonsäuredimethylester nach De Kayser et. al. (J.Org.Chem. 53, 4859-4862,1988) hergestellt. 3 g Monomer werden wie in Beispiel 1 polymerisiert. Es wird eine 2%-ige Lösung in THF hergestellt und zur Beschichtung, wie in Beispiel 3 beschrieben verwendet.

### Beispiel 28:

### Beschichtung eines medizinischen Implantats mit einem Polymer.

Es werden 1 ml des Methylenmalonsäuredimethylesters aus Beispiel 26 wie bei Lescure et al. (Pharm. Research 11(9), 1270-1277 (1994) durch tropfenweise Zugabe in 100 ml 1%iger Dextranlösung (MW = 70.000 Sigma) bei pH = 5.5 unter rühren polymerisiert. Die erhaltenen Nanopartikel werden 5 mal durch Zentrifugation gegen Wasser gewaschen, lyophilisiert und aus dem Lyophilisat eine 1%ige Polymerlösung in Methylenchlorid hergestellt und zur Beschichtung, wie in Beispiel 3 verwendet.

### Beispiel 29:

### Beschichtung eines medizinischen Implantats mit einem Polymer

Es werden 1 ml des Butycyanoacrylat durch tropfenweise Zugabe in 100 ml 1 %iger Dextranlösung (MW = 70.000 Sigma) bei pH = 2.5 unter rühren polymerisiert . Die erhaltenen Nanopartikel werden 5 mal durch Zentrifugation gegen Wasser gewaschen, lyophilisiert und aus dem Lyophilisat eine 1 %ige Polymerlösung in Methylenchlorid hergestellt und zur Beschichtung, wie in Beispiel 3 verwendet.

## Patentansprüche

1. Stent, welcher aus einem Träger besteht, der mit einem Polymeren oder einem Polymergemisch beschichtet ist, **dadurch gekennzeichnet, daß** das Polymergemisch einen Polycyanacrylsäureester oder einen Polymethylenmalonsäureester enthält.

2. Stent gemäß Anspruch 1, **dadurch gekennzeichnet, daß** der Träger aus Metall oder einem Polymeren besteht.

3. Stent gemäß einem der vorangegangenen Ansprüche, **dadurch gekennzeichnet, daß** die Beschichtung Polymere aus Cyanacrylatbutylester enthält.

4. Stent gemäß einem der Ansprüche 1 oder 2, **dadurch gekennzeichnet, daß** die Beschichtung aus Polycyanacrylsäureester und mindestens einem weiteren Polymeren besteht.

5. Stent gemäß Anspruch 4, **dadurch gekennzeichnet, daß** in der Polymerbeschichtung Substanzen enthalten sind, die den Abbau des Polymeren beeinflussen.

6. Stent gemäß Anspruch 5, **dadurch gekennzeichnet, daß** die Beschichtung Kalziumcarbonat enthält.

7. Stent gemäß Anspruch 4, **dadurch gekennzeichnet, daß** mindestens eines dieser weiteren Polymere aus einer der nachfolgend aufgeführten Substanzgruppen stammt: Proteine (insbesondere Albumin, Gelatine, Fibrinogen, Fibrin, Hirudin, Heparin, Collagen, oder Immunoglobuline) sowie deren Derivate (insbesondere quervernetzte Polypeptide, Konjugate von Proteinen mit Polyethylenglykolen und anderen Polymeren), Pseudopolyaminosäuren, Stärke, oder Stärkederivate, Chitin, Chitosan, Pektin, Polymilchsäure, Polyglykolsäure, Polyhydroxybuttersäure, Polyester, Polycarbonate, Polyamide, Polyphosphazene, Polyvinylalkohol, Polyaminosäuren, Poly-ε-caprolacton, Polyorthoester, Polyurethane, Polyharnstoff, Polyethylenterephtalat, Polymethylenmalonsäureester.

8. Stent gemäß Anspruch 4, **dadurch gekennzeichnet, daß** die aufgebrachte polymere Schicht mindestens einen Weichmacher enthält.

9. Stent gemäß Anspruch 8, **dadurch gekennzeichnet, daß** der Weichmacher ein nichtionisches Tensid ist, insbesondere Nonylphenoxy-Polyethylenoxid, Octoxynol oder Poloxamere.

10. Sterile Lösung eines Polymergemisches in einem speziellen Inkubationsgefäß zur Herstellung von Stents gemäß einem der Ansprüche 1 bis 9.

11. Verwendung von Polymeren aus Cyanacrylaten und/oder Methylenmalonsäureestern zur Beschichtung von Stents.

12. Verfahren zur Herstellung von Stents gemäß Anspruch 1, **dadurch gekennzeichnet, daß** der Stent-Träger in eine Lösung eingetaucht wird, die Polymere aus Cyanacrylat und/oder Methylenmalonsäureester enthält, und dann aus dieser Lösung herausgezogen wird.

13. Verfahren gemäß Anspruch 12, **dadurch gekennzeichnet, daß** die Lösung neben den Polymeren aus Cyanacrylat und/oder Methylenmalonsäureester weitere Polymere enthält.

14. Verfahren zur Herstellung eines mit Polymer beschichteten Stents unter Verwendung einer sterilen Lösung gemäß Anspruch 10.

## Claims

1. Stent which consists of a support coated with a polymer or a polymer mixture, **characterized in that** the polymer mixture contains a polycyanoacrylic acid ester or a polymethylene malonic acid ester.

2. Stent according to Claim 1, **characterized in that** the support consists of metal or a polymer.

3. Stent according to either of the preceding claims, **characterized in that** the coating contains polymers of cyanoacrylate butyl ester.

4. Stent according to either of Claims 1 and 2, **characterized in that** the coating consists of polycyanoacrylic acid ester and at least one further polymer.

5. Stent according to Claim 4, **characterized in that** substances which influence the degradation of the polymer are contained in the polymer coating.

6. Stent according to Claim 5, **characterized in that** the coating contains calcium carbonate.

7. Stent according to Claim 4, **characterized in that** at least one of these further polymers originates from one of the following substance groups: proteins (in particular albumin, gelatin, fibrinogen, fibrin, hirudin, heparin, collagen or immunoglobulins) and their derivatives (in particular crosslinked polypeptides, conjugates of proteins with polyethylene glycols and other polymers), pseudopolyamino acids, starch, or starch derivatives, chitin, chitosan, pectin, polylactic acid, polyglycolic acid, polyhydroxybutyric acid, polyester, polycarbonates, polyamides, polyphosphazenes, polyvinyl alcohol, polyamino acids, poly-ε-caprolactone, polyorthoester, polyurethanes, polyurea, polyethylene terephthalate, and polymethylene malonic acid ester.

8. Stent according to Claim 4, **characterized in that** the applied polymer layer contains at least one softener.

9. Stent according to Claim 8, **characterized in that** the softener is a nonionic surfactant, in particular nonylphenoxy polyethylene oxide, octoxynol or poloxamers.

10. Sterile solution of a polymer mixture in a special incubation vessel for the production of stents according to one of Claims 1 to 9.

11. Use of polymers of cyanoacrylates and/or methylene malonic acid esters for coating stents.

12. Process for the production of stents according to Claim 1, **characterized in that** the stent support is immersed in a solution containing polymers of cyanoacrylate and/or methylene malonic acid ester, and is then withdrawn from this solution.

13. Process according to Claim 12, **characterized in that**, in addition to the polymers of cyanoacrylate and/or methylene malonic acid ester, the solution contains further polymers.

14. Process for the production of a polymer-coated stent using a sterile solution according to Claim 10.

## Revendications

1. Stent constitué d'un support revêtu d'un polymère ou d'un mélange de polymères, **caractérisé en ce que** le mélange de polymères contient un poly(ester d'acide cyanoacrylique) ou un poly(ester d'acide méthylène-malonique).

2. Stent selon la revendication 1, **caractérisé en ce que** le support est constitué de métal ou d'un polymère.

3. Stent selon l'une des revendications précédentes, **caractérisé en ce que** le revêtement contient des polymères de butylester cyanoacrylique.

4. Stent selon l'une des revendications 1 ou 2, **caractérisé en ce que** le revêtement consiste en poly(ester cyanoacrylique) et au moins un autre polymère.

5. Stent selon la revendication 4, **caractérisé en ce que** le revêtement polymère contient des substances agissant sur la dégradation du polymère.

6. Stent selon la revendication 5, **caractérisé en ce que** le revêtement contient du carbonate de calcium.

7. Stent selon la revendication 4, **caractérisé en ce qu'**au moins un des autres polymères provient de l'un des groupes de substances ci-après: des protéines (en particulier de l'albumine, des gélatines, du fibrinogène, de la fibrine, de l'hirudine, de l'héparine, du collagène ou des immunoglobulines) ainsi que leurs dérivés (en particulier des polypeptides à réticulation transversale, des conjugués de protéines avec du polyéthylène glycol et d'autres polymères), des pseudo-polyaminoacides, des amidons ou des dérivés d'amidon, de la chitine, du chitosan, de la pectine, de l'acide polylactique, de l'acide polyglycolique, de l'acide polyhydroxybutyrique, des polyesters, des polycarbonates, des polyamides, des polyphosphazènes, du poly(alcool vinylique), des polyaminoacides, de la poly-ε-caprolactone, des polyorthoesters, des polyuréthannes, de la polyurée, du polyéthylène téréphtalate, du poly(ester d'acide méthylène-malonique).

8. Stent selon la revendication 4, **caractérisé en ce que** la couche de polymère appliquée contient au moins un plastifiant.

9. Stent selon la revendication 8, **caractérisé en ce que** le plastifiant est un agent tensioactif non ionique, en particulier de l'oxyde de nonylphénoxy-polyéthylène, de l'octoxynol ou des poloxamères.

10. Solution stérile d'un mélange de polymères dans un récipient spécial d'incubation pour la préparation de stents selon l'une des revendications 1 à 9.

11. Utilisation de polymères de cyanoacrylates et/ou d'esters d'acide méthylène-malonique pour le revêtement de stents.

12. Procédé de préparation de stents selon la revendication 1, **caractérisé en ce que** le support du stent est plongé dans une solution contenant les polymères de cyanoacrylates et/ou d'esters d'acide méthylène-malonique, puis est retiré de cette solution.

13. Procédé selon la revendication 12, **caractérisé en ce que** la solution, outre les polymères de cyanoacrylates et/ou d'esters d'acide méthylène-malonique, contient d'autres polymères.

14. Procédé de préparation d'un stent revêtu de polymère en utilisant une solution stérile selon la revendication 10.
